# EUROPEAN PATENT APPLICATION

(11) **EP 4 645 331 A1**
(43) Date of publication of application: **05.11.2025**
(21) Application number: 24382487.7
(22) Date of filing: 03.05.2024
(51) Int. Cl.: G16H 30/20

(54) **A METHOD AND SYSTEM FOR GENERATING ENCRYPTION AND DECRYPTION KEYS FOR MEDICAL IMAGES**

(71) Applicant: Universidad de Las Palmas de Gran Canaria, 35001 Las Palmas de Gran Canaria (ES)
(72) Inventor: SANTANA QUINTANA, Gabriel, 35018 Las Palmas de Gran Canaria (ES); CARRASCO HERNÁNDEZ, Eduardo Jorge, 35415 Arucas (ES); MELIÁN ÁLAMO, José María, 35019 Las Palmas de Gran Canaria (ES); GUERRA HERNÁNDEZ, Raúl Celestino, 35400 Arucas (ES); LÓPEZ SUÁREZ, Sebastián Miguel, 35011 Las Palmas de Gran Canaria (ES); MEDINA HERNÁNDEZ, Selenia María, 35110 Santa Lucía de Tirajana (ES)
(74) Representative: ZBM Patents - Zea, Barlocci & Markvardsen

(57) **Abstract**

There is provided an encryption key generation system and method for providing an encryption key and a decryption key to encode and encrypt an input set of medical images, embedded in a DICOM cube. Furthermore, a system and method for compressing and encrypting an input DICOM cube, and a system and method for decompressing and decrypting an input compressed and encrypted DICOM cube are also provided.

## Description

### FIELD OF DISCLOSURE

The present disclosure is related to Digital Imaging and Communications in Medicine.

### BACKGROUND

A large number of images are acquired daily during clinical practice, many of them being acquired sequentially producing three-dimensional data cubes (using a standard known as DICOM). This images are obtained using, for example, computed tomography (CT) and magnetic resonance imaging (MRI) devices. Each of these three-dimensional data cubes are usually constructed from hundreds of two-dimensional images that are stacked in a specific sequence to form a coherent three-dimensional representation of a human body or a specific part of one. The storage and transmission of such a large amount of data is an expensive and time-consuming process. Within the clinical environment, medical images have to be replicated for security and their access need to be restricted. Furthermore, both data integrity and security have to be ensured at all times. Therefore, there is a need for optimizing storage and transmission of medical images in such systems.

Compression has been applied in the past to medical image data cubes. In particular, video compressors, which are designed to take advantage of both spatial and temporal redundancies. However, video compressors based on motion prediction are not optimal to be applied to medical images since, unlike conventional video, wherein motion is generally evident, volumetric medical images show subtle changes and anatomical structures tend to change more by deformation than by motion. This makes it difficult to efficiently apply motion prediction techniques (such as, for example, MPEG type compressions), designed for compressing traditional videos, on volumetric medical images. In addition, standard video compression may not be sufficiently accurate to detect and preserve subtle variations between consecutive medical images, leading to a possible loss of clinically valuable information.

Therefore, there is a need for a new server and client architecture to overcome the limitations of the prior art solutions.

### SUMMARY

In a first aspect, the present disclosure provides an encryption key generation system configured to provide an encryption key and a decryption key to encode and encrypt an input set of at least two medical images, embedded in a DICOM cube. The encryption key generation system comprises:
- an input interface configured to receive at least one input DICOM cube; and
- a processor configured to perform a method comprising:
   - providing an auto-encoder neural network comprising an encoder and a decoder, wherein the encoder is configured to map an input medical image into a bitstream, and the decoder is configured to reconstruct the input medical image from the bitstream;
   - training the auto-encoder neural network with a training data set comprising at least a plurality of medical images of an input DICOM cube, the training comprising:
      - mapping each medical image into a respective bitstream; and
      - reconstructing each mapped medical image from the corresponding mapped bitstream;
      wherein the mapping and the reconstructing are performed using a loss function depending at least on the inter-image entropy of the DICOM cube, the loss function comprising penalizing variability among the bits that make up the bitstream when it exceeds a predetermined threshold, each of the bitstreams having a lower dimension than the dimension of the respective medical image, and the training resulting in a set of weights of the auto-encoder neural network; and

   - providing an encryption key of the encoder, the encryption key comprising a first portion of the resulting set of weights;
   - providing a decryption key of the decoder, the decryption key comprising a second portion of the resulting set of weights.

By using the proposed system when managing medical images within a medical data system, a compression and encryption of the medical images may be achieved using the provided encryption and decryption keys. Furthermore, a reduction of data storage and transmission requirements may also be achieved, while increasing data security. This may also allow using medical images in areas such as telemedicine, in a secure and fast way. Furthermore, the proposed system may also allow storing medical images in the cloud in a secure way or performing clinical studies in collaboration between different medical centers (for example, different hospitals).

A DICOM cube as presently disclosed may be defined as a set of medical images (two or more) of the same human body, the set of medical images stored within the cube using the DICOM standard and each medical image embodied within a DICOM file. DICOM stands for "Digital Imaging and Communications in Medicine", which is the international standard for the transmission, storage, display and printing of medical images in digital format. The medical images may be, for example, X-rays, magnetic resonance imaging (MRI) scans, computed tomography (CT) scans, and positron emission tomography (PET), among others. This type of medical images comes usually in a set of at least 200 or 300 medical images (for example, a CT scan of the abdomen area of a human body). Other sets of medical images can reach quantities of up to 1800 medical images within the set (for example, a CT scan of a leg of a human body). The separation in real life between medical images is usually less than 2,5mm in between medical images. An example may be a resolution of a CT scan performed to detect Idiopathic pulmonary fibrosis in the lungs of a patient may be set to 0,2mm between medical images.

A DICOM cube comprises a header part and a payload part. The DICOM header part comprises metadata of the DICOM file including information such as, for example, a patient's name, patient identifier, image modality, date and time of acquisition, details of the equipment used to acquire the image, and technical parameters of the study. The metadata may include information such as the position of the image in a series of images, its orientation, i.e., axial, sagittal, coronal, etc., the acquisition parameters, e.g., slice thickness, slice spacing or the distance between slices, wherein a slice is one of several images representing a 3D type of image from a human body, the images found within the DICOM cube. The payload part of the DICOM file comprises the image itself, stored in digital form.

In the type of medical images found within a DICOM cube, each consecutive medical image within a DICOM cube may comprise relatively little or no difference with respect to previous and following medical image or images. New elements within the medical image may appear compared to previous and following medical image(s). Furthermore, other elements in the medical image already present in previous medical images may remain or may be enlarged along consecutive images.

The compression and encryption of a DICOM cube may be performed by using an auto-encoder neural network.

Once the auto-encoder is trained, the encoder of the autoencoder neural network may be further configured to generate an encrypted and compressed DICOM cube based on an input DICOM cube. The compressed DICOM cube is embodied in a bitstream, the bitstream having a lower dimension than the original DICOM cube, i.e., occupying less bits than the original DICOM cube. Thus, the decoder of the autoencoder neural network may be further used to decompress and decrypt the input DICOM cube based on a bitstream (i.e., a compressed DICOM cube).

Furthermore, it is not possible to correctly decompress a compressed DICOM cube without the decoder corresponding to the same auto-encoder neural network as the encoder. Furthermore, by virtue of the compressed DICOM cube being embodied in a bitstream, the compressed DICOM cube is an encrypted version of the original DICOM cube.

In order to generate an encryption and decryption key, in examples, the auto-encoder neural network is trained using a plurality of DICOM cubes as an input training data set. Advantageously, each DICOM cube may comprise, for example, a plurality of images of a different patient, is used to train the auto-encoder neural network, the training resulting in a set of weights of the auto-encoder neural network. Said training may be performed using, for example, hundreds or thousands of different DICOM cubes.

When the training is performed, the resulting set of weights comprises a first portion used as an encryption key of the encoder of the auto-encoder neural network. Furthermore, a second portion of the set weights is used as a decryption key of the decoder of the auto-encoder neural network. In an example, the first portion of the set of weights may correspond to the weights of the encoder, and the second portion of the set of weights may correspond to the weights of the decoder.

More precisely, the training is performed by mapping a bitstream for each medical image within an input DICOM cube and reconstructing each medical image of the input DICOM cube from the corresponding mapped bitstream, the reconstruction being performed using a loss function which depends at least on the inter-image entropy of the DICOM cube, the loss function comprising penalizing variability among the bits that make up the bitstream when it exceeds a predetermined threshold. When compressing a medical image, the main goal may be to reach a high compression ratio. This can be achieved by minimizing the entropy between images when mapping a medical image, by comparing the original medical image with the mapped medical image in the form of a bitstream.

This comparison is performed by the loss function, which depends at least on the inter-image entropy of the DICOM cube. Since the medical images corresponding to the same DICOM cube are highly correlated, the compression and decompression process performed in the training of the auto-encoder neural network takes into account redundancies which are present in several consecutive or nearby medical images within the DICOM cube, when the entire plurality of medical images of the same DICOM cube are compressed and decompressed as a whole for the same DICOM cube. The inter-image entropy of the DICOM cube may further comprise the entropy of the same pixel position of different medical images. Furthermore, the loss function also comprises penalizing the variability among the bits that make up the bitstream when it exceeds a predetermined threshold. Advantageously, the loss function drives the composition of each bitstream of each medical image to be as homogeneous as possible. As a result of this, the loss function forces to minimize to a certain degree (defined by the loss function itself) the entropy between the medical images of the DICOM cube when the training is performed, which further results in an increase in the compression ratio of the medical images when training the auto-encoder neural network (and in the subsequent performance of the auto-encoder neural network once it has been trained).

In some examples, a second loss function is used at when the reconstruction of the mapped medical image is performed. This second loss function is aimed to minimize the error of the compression while reaching a high compression rate of the mapped medical image (in the form of a bitstream) at the same time. The error of the decompression may be defined as a quantification of how similar the decompressed medical image is to the original medical image. The more different the decompressed medical image is from its original medical image, the higher the error.

Furthermore, in some examples a randomization step may be performed during the training of the auto-encoder neural network. In a DICOM cube, for example, a DICOM cube comprising images from a CT scan, each consecutive medical image within the DICOM cube has subtle differences from previous and following medical image(s). Furthermore, DICOM cubes comprising medical images from different human bodies may also present subtle changes. Therefore, different trainings of the auto-encoder neural network, each training using a different set of training DICOM cubes, result in different sets of weights which may be very similar between each other. Therefore, randomization techniques may be applied in order to avoid the possibility of using decryption keys to decrypt compressed DICOM cubes previously encrypted with an unrelated encryption key; in other words, randomization provides a more robust encryption. By performing said randomization step, even if the exact same set of DICOM cubes is used to perform a training of the auto-encoder neural network twice, the two resulting sets of encryption and decryption keys may not be interchangeable, thus achieving a more robust encryption.

This way, by using techniques that allow randomizing different stages of the training process, it is possible to obtain trained neural networks with similar accuracy but with completely different weights. In some examples, said randomization techniques may comprise one or more of:
- Random weight initialization (i.e., He initialization, Glorot, Xavier, or random distributions);
- Reordering of the training data at each epoch, altering the training and test data sets, or changing the size of the input batches;
- Regularization techniques such as: Dropout, L1 Regularization, L2 Regularization, or Noise Injection.

These randomization techniques may be used for avoiding the overfitting of a neural network. Overfitting occurs when a machine learning algorithm learns the training dataset too well, including the noise and fluctuations present in that specific dataset. As a result, the algorithm may perform well on the training dataset but may fail to generalize effectively to new, unseen data.

When the auto-encoder neural network is trained, an encryption key of the encoder and a decryption key of the decoder are provided. The provided encryption key may be used to compress and encrypt an input DICOM cube, for example, before storing it in a server. Furthermore, the provided decryption key may be used to decompress and decrypt a compressed and encrypted DICOM cube (embedded in a bitstream). Advantageously, by providing the decryption key to a user authorised to decompress a specific DICOM cube, the specific compressed and encrypted DICOM cube (embodied in a bitstream), may be, for example, subsequently downloaded by the authorized user and decompressed locally at the authorized user's terminal.

While compression can be very useful for handling large amounts of data, in the presently disclosed system it is also crucial to ensure the quality of the decompressed medical images, so that an expert can make an accurate diagnosis when observing those decompressed medical images. The compression method and ratio may therefore be based on clinical and technical considerations. Nowadays, single medical images (ultrasound, mammograms, etc.), and sets of medical images embedded in DICOM cubes (such as, for example, CT scans, MRI, or PET), are compressed and stored independently within a DICOM file. Therefore, each image is compressed separately, which does not allow to take advantage of redundancies between the different images.

The presently disclosed system does take advantage of the redundancies found within DICOM cubes, i.e., the inter-image entropy of the DICOM cube, allowing to reduce the amount of data to be stored. Furthermore, the presently disclosed system allows protecting patient data from being stolen or manipulated and allows an efficient management of the time and use of resources when managing patient data. This way, healthcare systems may protect the privacy and security of patient's data found within DICOM cubes. Also, the presently disclosed system may also achieve the encryption of DICOM cubes.

In some examples, the auto-encoder neural network may comprise at least one recurrent layer. The auto-encoder neural network comprising a recurrent layer does not necessarily imply that the auto-encoder neural network is considered recurrent. In particular, sequences of images, such as time-series data from MRI or CT scans, can be processed using recurrent auto-encoder neural networks to recognize temporal dependencies, to be used for tasks like, for example, disease progression prediction.

In some examples, the auto-encoder neural network may comprise a Long short-term memory (LSTM) neural network, which is an example of neural network comprising at least one recurrent layer. LSTM neural networks are designed to handle sequential data by capturing long-term dependencies and are commonly associated with natural language processing. However, since LSTMs are particularly effective when dealing with sequences of data where temporal dependencies are crucial, they may also capture relationships between consecutive images.

In some examples, the LSTM neural network may comprise a Convolutional Long Short-Term Memory, ConvLSTM, layer. By processing the medical images of a DICOM cube in a sequential manner, the ConvLSTM layer can identify which areas of successive images evolve and which ones remain constant. In this way, when being trained, the auto-encoder neural network learns to recognize patterns and redundancies throughout the medical images of a DICOM cube, which subsequently allows for efficient compression. Furthermore, the resulting images compressed and further decompressed using the present example, i.e., a LSTM neural network comprising a ConvLSTM layer, may also advantageously retain medical information relevant to be further examined by, for example, a doctor.

Alternatively, the LSTM neural network may comprise at least a time distributed layer and a convolutional layer. Advantageously, the LSTM neural network may be particularly suitable for tasks where both spatial dependencies and temporal dependencies (i.e., dependencies along several images of the DICOM cube) occur. In the case of medical images within the same DICOM cube, the spatial dependencies may occur within the same medical image. Furthermore, the temporal dependencies occur within a sequence of images that make up the DICOM cube.

A time distributed layer is characterized by applying the same operation to each element of a data sequence. Traditionally, this type of layer is used in contexts wherein the data has a temporal dimension. However, the "temporal dimension" usually associated with this layer is replaced by the "physical depth" represented in each medical image of the set of medical images of the DICOM cube. A time distributed layer can apply consistent operations across a set of medical images of a DICOM cube, thus facilitating the analysis and processing of each medical image of the set of medical images in a uniform manner, ensuring consistency on the treatment along each medical image.

More specifically, this type of layer is particularly efficient for capturing the spatial features within each individual medical image, such as edges, textures and patterns, thanks to the convolutions found within the time distributed layer. By applying convolutions uniformly across the different medical images of the DICOM cube, the network is able to identify spatial features that remain consistent throughout the set of medical images of a DICOM cube. This consistency is the result of a large amount of redundant information found within the set of medical images of a DICOM cube, such as, for example, non-changing parts of the medical images which correspond to organs or bones with a similar shape and/or size along part or all of the set of medical images. Thus, the homogeneous application of convolutions makes it easier to identify said redundancies within a single image, and at the same time also allows detecting redundancies throughout the entire DICOM cube.

By treating each image in the data cube independently, the time distributed layer focuses more on extracting and understanding detailed spatial features of each image, although these may not be as efficient in capturing the relationships between successive images. Furthermore, a time distributed layer may be useful for sets of medical images that have a very high correlation between each medical image, such as, for example, a CT scan. Furthermore, time distributed layers are computationally lighter than ConvLSTM layers, thus requiring less computational resources.

Therefore, either type of neural networks, LSTM comprising a ConvLSTM layer or LSTM comprising a time distributed layer and a convolutional layer, significantly improves the system's ability to process and understand information contained in medical data, maximizing efficiency in the identification of relevant patterns and eliminating redundancies.

ConvLSTM layers may be more suitable to exploit the three-dimensional relationships within the DICOM cube (i.e., inter-image relationships) and the evolution of spatial information throughout the DICOM cube.

On the other hand, the combination of one or more time distributed layers with one or more convolutional layers would be the best choice in the case of wanting to maintain consistency across the set of medical images within the DICOM cube.

In some examples, the auto-encoder neural network may comprise a Transformers-type neural network. Transformers-type neural networks are a type of neural network architecture important in the field of natural language processing (NLP). A distinctive feature of Transformers is their attentional mechanism, which allows them to assign different levels of importance to different parts of the input data for the performance of specific tasks. Furthermore, this architecture is particularly effective at handling long sequences of data, establishing complex relationships between them. This makes them especially valuable when handling medical images, wherein the context and the relationship between large amounts of data are important. Furthermore, they can identify the most relevant parts of an image to preserve its quality and essential information, thus facilitating efficient compression without generating a significant loss of important detail.

In some examples, the Transformers-type neural network may comprise a Vision Transformers ViT neural network. Transformer architectures adapted for image processing are known as Vision Transformers, or ViT. These models approach image processing by treating them as sequences of "patches", an approach that resembles how sequences of data are handled in natural language processing. Each patch of an image is associated with positional coordinates, a technique that allows the model to understand and maintain the relative spatial location of each patch within the image, which is crucial for capturing the full spatial structure. What distinguishes ViTs from other neural networks is the use of attentional mechanisms, which allow them to capture and analyze complex relationships between these patches and their relative positions. This ability to handle large sequences of data, focusing on the interconnections between different segments of an image and their spatial arrangement, allows to detect detailed spatial features, thus identifying complex patterns and details within an image, considering both the individual content of the individual content of patches and their spatial context in the overall image.

In some examples, a compression and encryption system may be provided, the system being configured to compress and encrypt an input DICOM cube. Furthermore, the system may comprise:
- an input interface configured to receive at least one input DICOM cube;
- an output interface configured to output at least one compressed and encrypted DICOM cube; and
- a processor configured to provide a compressed and encrypted DICOM cube, embodied in a bitstream, based on an input DICOM cube, by:
   - receiving an input DICOM cube;
   - compressing and encrypting the input DICOM cube using an encryption key provided by an encryption key generation system as previously disclosed.

The compression and encryption system may be embedded in a server (for example, a PACS server) of a medical data system, the server being able to compress and encrypt an incoming DICOM cube sent from a device (for example, a CT device) and further store it in, for example, a database.

In some examples, a decompression and decryption system may be provided, the system being configured to decompress and decrypt an input compressed and encrypted DICOM cube. Furthermore, the system may comprise:
- an input interface configured to receive at least one input compressed and encrypted DICOM cube, embodied in a bitstream;
- an output interface configured to output at least one decompressed and decrypted DICOM cube; and
- a processor being configured to provide a decompressed and decrypted DICOM cube, based on an input compressed and encrypted DICOM cube, by:
   - receiving an input compressed and encrypted DICOM cube;
   - decompressing and decrypting the input compressed and encrypted DICOM cube using a decryption key provided by an encryption key generation system as previously disclosed.

The decompression and decryption system may be embedded in, for example, a user terminal which may receive a compressed and encrypted DICOM cube from a server (for example, a PACS server) of a medical data system, and may further decompress and decrypt it using a key previously provided to the user terminal by, for example, the same server from which the compressed and encrypted DICOM cube is sent. This way, a server may be able to control the access to the medical images of specific DICOM cubes, by specific users of a medical data system.

In some examples, the encryption key generator system previously disclosed may be embedded within the compression and encryption system, and/or embedded within the decompression and decryption system.

In some aspects, the disclosure presents a computer program product comprising instructions which, when the program is executed by a computer, cause the computer to carry out the steps of a method for generating an encryption and a decryption key, a method for compressing and encrypting an input DICOM cube, or a method for decompressing and decrypting an input compressed and encrypted DICOM cube, according to the present disclosure.

The above description is only an overview of the technical solution of the present invention. In order to understand the technical means of the present invention more clearly, it can be implemented in accordance with the content of the specification, and in order to make the above and other objectives, features and advantages of the present invention more obvious and understandable. In the following, the preferred embodiments are cited in conjunction with the drawings, and the detailed description is as follows.

### DESCRIPTION OF THE FIGURES

Figure 1 depicts an example of a medical data system comprising a compression and encryption system, and a decompression and decryption system, according to the present disclosure.
Figure 2A depicts an example of a structure of a DICOM file having an uncompressed DICOM cube embedded within, according to the present disclosure.
Figure 2B depicts an example of a structure of a DICOM file, having a compressed DICOM cube embedded within, according to the present disclosure.
Figure 3 depicts an example of a method for training an auto-encoder neural network, according to the present disclosure.
Figure 4 depicts and example of a method for compressing, encrypting, decompressing and decrypting a DICOM cube according to the present disclosure.

### DETAILED DESCRIPTION

In order to further illustrate the technical means and effects of the present invention to achieve the intended purpose of the invention, the specific implementation, structure, features and effects of the present invention will be described in detail below with reference to the accompanying drawings and preferred embodiments.

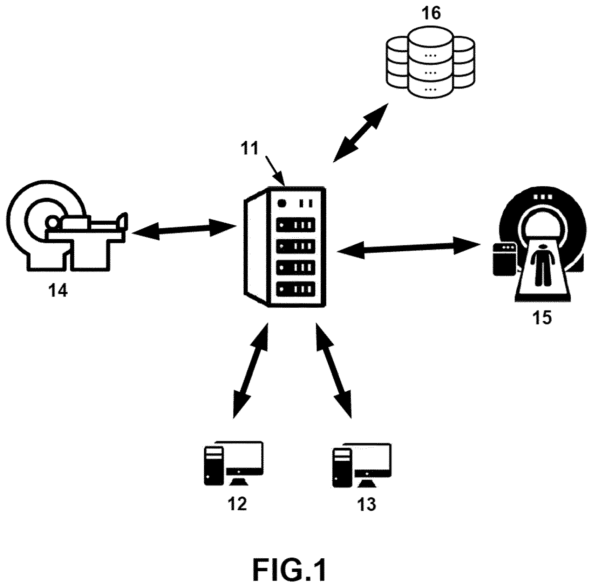

Figure 1 shows an example of a PACS medical data system 1 comprising a PACS server 11, a database 16 connected to the PACS server 11, two user terminals 12 and 13 connected to the PACS server 11, a Computed Tomography Scan device 14, also referred to as CT device 14, and a Magnetic Resonance Imaging device, also referred to as MRI device 15.

A PACS server 11 is a server to which users of the PACS medical data system 1 connect to obtain or modify medical images of interest. Therefore, the user terminals 12, 13 are connected to the PACS server 11. Furthermore, the devices 14, 15 are also connected to the PACS server 11. In this example, the PACS server 11 is further connected to a database 16, which comprises a storage system responsible for storing the medical images and other medical related data. These type of databases 16 are usually high-capacity redundant systems, ensuring that data is protected and available at all times. They may consist of, for example, high-capacity hard disks, RAIDs or cloud storage solutions.

In the PACS medical data system 1, each device 14, 15 performing medical tests resulting in medical images sends the resulting medical images to the PACS server 11, to store the medical images within the database 16. Furthermore, each user of the PACS medical data system 1 (e.g. doctors, nurses or other medical related professionals) has a user terminal 12, 13 from which he/she connects to the PACS server 11 to retrieve or perform other operations with medical images stored within the database 16. Therefore, within such PACS medical data system 1, medical professionals view the medical images as a client connected to the PACS server 11 (i.e., they can download the medical images or access to them while connected to the PACS server 11). The users 12, 13 or the devices 14, 15 connected to the PACS server 11 can send queries to the PACS server 11 in order to perform operations.

The PACS server 11 uses the DICOM standard to manage DICOM files. Furthermore, the DICOM cubes as presently disclosed are embedded in DICOM files.

A DICOM file, is an encapsulation of descriptive data (i.e., a metadata part) and image data (i.e., a payload part), in a single file.

The metadata part within the DICOM file contains information on a patient, study, series and image to which it refers, as well as technical acquisition parameters and details of the equipment used. For example: type of equipment or machine used to acquire the image, e.g. "PET" for Positron emission tomography, or "CT" for Computed Tomography, or a textual description of the study performed which resulted on the DICOM file. The metadata part is organized in a structure of pairs, label-value, known as "tags".

The payload part is the part of the DICOM file that contains the data corresponding to tests or measurements of the patient, which in the present disclosure, is the medical images within the DICOM cube.

In the present example, when a DICOM cube is compressed, it is embedded in a DICOM file.

The DICOM file embedding the compressed DICOM cube comprises a metadata part with a specific tag called "Transfer Syntax UID" that indicates how the medical images within have been compressed. This specific tag ensures that the system receiving the image (for example, user terminal 12 or 13), or any other system manipulating the file, can decode and display the image correctly. Therefore, the Transfer Syntax UID allows interoperability and correct visualization of medical images when using the DICOM standard.

In this example, a compressed DICOM cube embedded in a DICOM file has a payload part comprising all the compressed medical images from the original DICOM cube. Before compressing the DICOM cube, the individual headers of each of the original medical images of the original DICOM cube may be redundant (for example, each header may have data related to the resolution or format of the image, the patient or time and place where the images were obtained). Therefore, the redundant metadata of each header of the plurality of medical images within the original DICOM cube may be erased, and they may be copied once in the metadata part of the compressed DICOM cube, thus saving data storage and further compressing the DICOM cube.

Furthermore, the medical images within the compressed DICOM cube contain a variety of private patient data including diagnostic and examination records. During storage, or the transmission of the DICOM file over a channel, the data within the file may be vulnerable to theft or unauthorized modification. Medical images are considered biomedical data and require protection (for example, privacy or protection against tampering or undue modification of the image). In this context, encryption of medical images in healthcare systems is essential to protect patient information. The time and computational resources consumed by encryption processes presents a significant challenge.

When users or devices of the PACS medical data system (both users and devices called DICOM clients from therein) try to access, modify, or write data of the medical images within the DICOM cubes stored in the database 16, the DICOM clients do it through requests to the PACS server 11. Some of the requests may comprise one or more of the following instructions: "STORE", "MOVE", "GET", "WRITE" and "FIND".

STORE is an operation used to store new DICOM cubes within the database 16. For example, DICOM cubes sent from devices such as CT scans 14 or MRI scans 15.

FIND is an operation that allows a DICOM client, such as a radiology workstation or an image viewer (for example, user terminals 12 or 13), to search for DICOM cubes within the database 16 or to search for specific information about patients, studies, series or images within the database 16. These search requests can be based on specific criteria such as patient name, study date or study type, among others.

GET allows a DICOM client to request that the PACS server 11 retrieves and sends specific studies, series or images of a patient from the database 16. This operation may be used when a user terminal 12, 13 wishes to access a medical image or DICOM cube that is not locally stored within the terminal 12, 13.

MOVE allows a DICOM client to get the PACS server 11 to transmit, or "move", specific studies, series or images from their current location within the database 16 to another DICOM client connected to the PACS medical data system 1. This operation is used when, for example, a study stored on the database 16 needs to be sent to a user terminal for further analysis or to another hospital department.

WRITE allows a DICOM client to modify the metadata associated with a specific study, series or medical image within the database 16. This operation is used to correct or update information within the DICOM image headers.

All of the previously disclosed instructions are sent to the PACS server 11, which manages each query and performs the requested operation.

In examples, user's queries are modified to allow the PACS server 11 to encode or to decode, i.e., compress and encrypt or decompress and decrypt, one or more DICOM cubes. In examples, the STORE function may be modified to include a new unified header. The new unified header may comprise common metadata information from a set of medical images of DICOM cube to be compressed. The new unified header stores, therefore, constant or common information for all the medical images in the set of medical images within the DICOM cube.
Furthermore, in some alternative examples to the example of figure 1, the PACS server 11 may be distributed in a cloud.

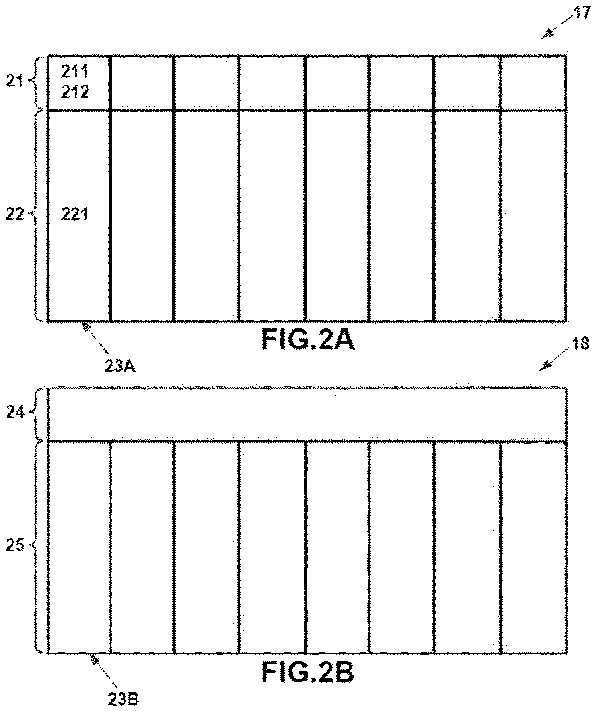

Figure 2A depicts an example of a structure of a DICOM file 17 comprising a DICOM cube embedded within, and figure 2B depicts an example of a structure of a DICOM file 18 comprising the DICOM cube of figure 2A after being compressed and encrypted, the compression and encryption being performed by the PACS server 11.

The DICOM file 17 of figure 2A comprises a plurality of images 23A, wherein each image 23A comprises a header part 21 and a payload part 22. Each header part 21 of each image 23A comprises general metadata 211 corresponding to all the medical images 23A stored within the DICOM cube, and a metadata part 212 corresponding to the corresponding image 23A. Furthermore, the payload part 22 of each image 23A comprises the data 221 corresponding to the image 23A itself in JPEG format.

In this example, when the compression and encryption method presently disclosed is used by the PACS system 1 of the example of figure 1, the PACS server 11 may compress and encrypt the DICOM cube embedded in the DICOM file 17 of figure 2A, using the first encryption key. In this example, figure 2B depicts the resulting structure of a DICOM file 18 comprising the compressed and encrypted DICOM file 17, the DICOM file 18 comprising compressed images 23B each corresponding to the images 23A of DICOM file 17. In this example, DICOM file 18 comprises a single header part 24 and a plurality of payload parts 25. The header part 24 comprises metadata which is common to all the compressed and encrypted images 23B found within the payload parts 25. This way, the size of DICOM file 18 is reduced from the size of DICOM file 17. In this example, a custom tag within the header 24, labelled as "Original Transfer Syntax UID", allows the PACS server 11 to know the format of any original medical image 23A (found in the payload part 221) in order to reconstruct the original DICOM cube of DICOM file 17 by decompressing the corresponding payload part 25 of a compressed medical image 23B. This may make it possible to obtain the original format of the images if necessary.

Furthermore, during the compression and encryption process, common metadata of the medical images (found in the header part 221) may also be identified, and further stored only once in the header part 24 of the DICOM file 18. This allows storing the original common metadata with as little information as possible.

During the compression process, the metadata that is unique for each medical image (found in the header part 211) is also extracted and stored in list tags (the Value Multiplicity (VM) tag defined in the DICOM standard, and found in the header part 24 of DICOM file 18). This way, special tags (already provided for in the DICOM file standard) that can store lists of data can be used to further reduce the size of the DICOM file 17 when compressing and encrypting a DICOM cube into the compressed and encrypted DICOM file 18.

An example of tags which may be unique for each medical image, and which may be included in these list tags is: SOP Instance UID, Instance Number, Image Position (Patient), Acquisition Time, Slice Thickness, or Slice Location.

Using a single header 24 with such DICOM file structure for the DICOM file 18 may ensure compatibility with a hospital's equipment or medical software already available.

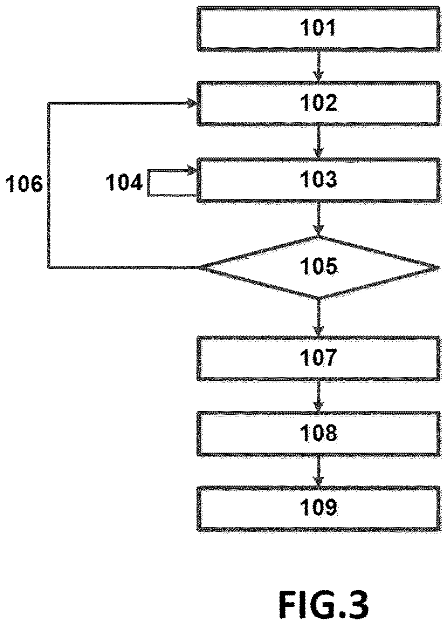

Figure 3 depicts a diagram of an example of the method for training an auto-encoder neural network, performed using the PACS medical data system 1 of figure 1, according to the present disclosure.

In step 101, the PACS server 11 obtains from the database 16 an auto-encoder neural network and a training data set of 500 DICOM cubes stored therein. The auto-encoder neural network comprises an encoder and a decoder, both being defined by a corresponding set of weights. When data is inputted in the auto-encoder neural network, the encoder compresses the input data into a lower-dimensional representation. Then, the decoder reconstructs the original data from this representation. In this example, a bitstream of 9 bits is used as a lower-dimensional representation of an inputted DICOM cube. Furthermore, the first DICOM cube is selected from the 500 DICOM cubes of the training data set.

Then, in step 102, the training of the obtained auto-encoder neural network begins, using the selected DICOM cube. In this example, the training is performed in a further terminal not shown. All of the medical images found within the selected DICOM cube are extracted from the selected DICOM cube to be used in the following step.

In step 103, the training of the auto-encoder neural network is performed iteratively (in a plurality of epochs 104) for each extracted medical image comprised in the selected DICOM cube. A first loss function is chosen to minimize the inter-image entropy of the DICOM cube. In this example, this first loss function is a standard deviation type function. Such standard deviation type function reduces the variability of the pixels of the input (i.e., mapped) medical image, by homogenizing the pixels of said image around a specific value. A second loss function is also chosen to quantify the difference between each input medical image and the corresponding reconstructed output medical image. In this example, the second loss function is a Mean Squared Error (MSE). For each of the medical images within the selected DICOM cube, the medical image is mapped into a bitstream using the encoder of the auto-encoder neural network. Furthermore, the medical image is reconstructed using the decoder of the auto-encoder neural network, from the corresponding mapped bitstream. The difference between the input medical image and the reconstructed medical image is computed using the chosen loss function. This measures how well the autoencoder is reconstructing the input medical image. Furthermore, the gradients of the loss with respect to the auto-encoder neural network parameters are calculated using backpropagation. This way, the error is propagated backwards through the auto-encoder neural network. Then, the auto-encoder neural network parameters (weights and biases) are updated using an optimization algorithm, such as stochastic gradient descent (SGD) or one of its variants (e.g., Adam). The goal of the backpropagation and the update of the parameters is to minimize the loss functions.

In step 105, if there are still DICOM cubes from the training set to be used to train the auto-encode neural network, a new DICOM cube is selected, and the training of the auto-encoder neural network (steps 102 to 105) is repeated by 106 with the new DICOM cube. When all the DICOM cubes from the training data set have been used to train the auto-encoder neural network, the method goes to step 107.

In step 107, as a result of the training performed on the auto-encoder neural network, a set of weights of the auto-encoder neural network is obtained. Then, in step 108, a first portion of weights corresponding to the encoder are selected from the set of weights of the auto-encoder neural network, the first portion of weights being stored within the database 16 as a first encryption key. Furthermore, in step 109, a second portion of weights corresponding to the decoder are selected from the set of weights of the auto-encoder neural network, the second portion of weights being sent to user terminals 12 and 13 as a first decryption key.

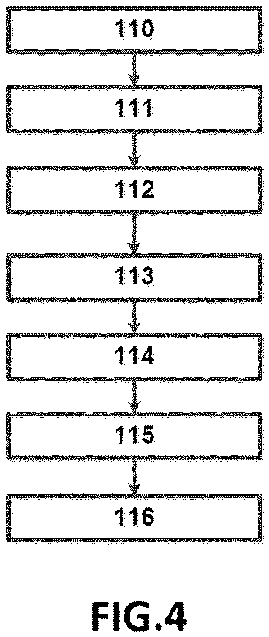

Figure 4 depicts a diagram of an example of a method for compressing, encrypting, decompressing and decrypting a DICOM cube, according to the present disclosure.

In step 110, the CT scan device 14 sends a query to PACS server 11, comprising a STORE instruction.

In step 111, the PACS server 11 handshakes with the CT scan device 14 and then the CT scan device 14 sends a new DICOM cube comprising a CT scan of a patient (comprising 1000 medical images) to the PACS server 11.

In step 112, the PACS server 11 compresses and encrypts the received DICOM cube using the first encryption key found in the database 16.

Then, in step 113, the compressed DICOM cube, embedded in a bitstream, is stored by the PACS server 11 on the database 16.

In step 114, user terminal 12 sends a query to the PACS server 11, the query comprising a GET instruction, specifying the name tag of the DICOM cube stored in the database 16 in step 113.

In step 115, the PACS server 11 retrieves the compressed and encrypted DICOM cube specified in the received GET instruction, and sends the retrieved compressed and encrypted DICOM cube to the user terminal 12.

In step 116, user terminal 12 receives the compressed and encrypted DICOM cube. The terminal 12 reads the header of the compressed DICOM cube and uses the first decryption key stored within itself to decompress DICOM cube, using header list in compressed DICOM cube to reconstruct all the medical images in an orderly manner.

## Claims

1. An encryption key generation system configured to provide an encryption key and a decryption key to encode and encrypt an input set of at least two medical images, embedded in a DICOM cube, the system comprising:
- an input interface configured to receive at least one input DICOM cube;
- a processor configured to perform a method comprising:
- providing an auto-encoder neural network comprising an encoder and a decoder, wherein the encoder is configured to map an input medical image into a bitstream, and the decoder is configured to reconstruct the input medical image from the bitstream;
- training the auto-encoder neural network with a training data set comprising at least a plurality of medical images of an input DICOM cube, the training comprising:
- mapping each medical image into a respective bitstream; and
- reconstructing each mapped medical image from the corresponding mapped bitstream;
wherein the mapping and the reconstructing are performed using a loss function depending at least on the inter-image entropy of the DICOM cube, the loss function comprising penalizing variability among the bits that make up the bitstream when it exceeds a predetermined threshold, each of the bitstreams having a lower dimension than the dimension of the respective medical image, and the training resulting in a set of weights of the auto-encoder neural network; and
- providing an encryption key of the encoder, the encryption key comprising a first portion of the resulting set of weights;
- providing a decryption key of the decoder, the decryption key comprising a second portion of the resulting set of weights.

2. An encryption key generation system according to claim 1, wherein the processor is further configured to train the auto-encoder neural network with a training data set comprising a plurality of DICOM cubes.

3. An encryption key generation system according to any of claims 1 or 2, wherein the training of the auto-encoder neural network further comprises applying a randomization step.

4. An encryption key generation system according to claim 3, wherein the randomization step comprises performing any of:
- random weight initialization; or
- adding at least one regularization layer to the auto-encoder neural network.

5. An encryption key generation system according to any of claims 1 to 4, wherein the auto-encoder neural network comprises at least one recurrent layer.

6. An encryption key generation system according to claim 5, wherein the auto-encoder neural network comprises a Long short-term memory LSTM network.

7. An encryption key generation system according to claim 6, wherein the LSTM network comprises a ConvLSTM layer or a combination of a time distributed layer and a convolutional layer.

8. An encryption key generation system according to any of claims 1 to 4, wherein the auto-encoder neural network comprises a Transformers-type neural network.

9. An encryption key generation system according to claim 8, wherein the Transformers-type neural network comprises a Vision Transformers ViT neural network.

10. A compression and encryption system configured to compress and encrypt an input DICOM cube, the system comprising:
- an input interface configured to receive at least one input DICOM cube;
- an output interface configured to output at least one compressed and encrypted DICOM cube; and
- a processor configured to provide a compressed and encrypted DICOM cube, embodied in a bitstream, based on an input DICOM cube, by:
- receiving an input DICOM cube;
- compressing and encrypting the input DICOM cube using an encryption key provided by an encryption key generation system according to any of claims 1 to 9.

11. A decompression and decryption system configured to decompress and decrypt an input compressed and encrypted DICOM cube, the system comprising:
- an input interface configured to receive at least one input compressed and encrypted DICOM cube, embodied in a bitstream;
- an output interface configured to output at least one decompressed and decrypted DICOM cube; and
- a processor being configured to provide a decompressed and decrypted DICOM cube, based on an input compressed and encrypted DICOM cube, by:
- receiving an input compressed and encrypted DICOM cube;
- decompressing and decrypting the input compressed and encrypted DICOM cube using a decryption key provided by a system according to any of claims 1 to 9.

12. A computer implemented method for generating an encryption and a decryption key to encode and encrypt an input set of at least two medical images, embedded in a DICOM cube, the method comprising:
- providing at least one input DICOM cube;
- providing an auto-encoder neural network comprising an encoder and a decoder, wherein the encoder is configured to map an input medical image into a bitstream, and the decoder is configured to reconstruct the input medical image from the bitstream;
- training the auto-encoder neural network with a training data set comprising at least a plurality of medical images of the input DICOM cube, the training comprising:
- mapping each medical image into a respective bitstream; and
- reconstructing each mapped medical image from the corresponding mapped bitstream;
wherein the mapping and the reconstructing are performed using a loss function depending at least on the inter-image entropy of the input DICOM cube, the loss function comprising penalizing variability among the bits that make up the bitstream when it exceeds a predetermined threshold, each of the bitstreams having a lower dimension than the dimension of the respective medical image, and the training resulting in a set of weights of the auto-encoder neural network; and
- providing an encryption key of the encoder, the encryption key comprising a first portion of the resulting set of weights;
- providing a decryption key of the decoder, the decryption key comprising a second portion of the resulting set of weights.

13. A computer implemented method for compressing and encrypting an input DICOM cube, the method comprising:
- providing at least one input DICOM cube;
- providing a compressed and encrypted DICOM cube, embodied in a bitstream, based on the input DICOM cube, by compressing and encrypting the input DICOM cube using an encryption key provided by performing the method according to claim 12.

14. A computer implemented method for decompressing and decrypting an input compressed and encrypted DICOM cube, the method comprising:
- providing at least one input compressed and encrypted DICOM cube, embodied in a bitstream;
- providing a decompressed and decrypted DICOM cube, based on an input compressed and encrypted DICOM cube by decompressing and decrypting the input compressed and encrypted DICOM cube using a decryption key provided by performing the method according to claim 12.

15. A computer program product comprising instructions which, when the program is executed by a computer, cause the computer to carry out the steps of a method according to any one of claims 12 or 13 or 14.
